# EUROPEAN PATENT SPECIFICATION

(11) **EP 1 217 978 B1**
(45) Date of publication and mention of the grant of the patent: **16.03.2005**
(21) Application number: 00957200.9
(22) Date of filing: 23.08.2000
(51) Int. Cl.: A61F 13/534, A61L 15/60

(54) **ABSORBENT STRUCTURE IN AN ABSORBENT ARTICLE**
ABSORBIERENDE STRUKTUR IN EINEM ABSORBIERENDEN ARTIKEL
STRUCTURE ABSORBANTE INTEGREE A UN ARTICLE ABSORBANT

(30) Priority: 30.08.1999 SE 9903072
(43) Date of publication of application: 03.07.2002
(73) Proprietor: SCA Hygiene Products AB, 405 03 Göteborg (SE)
(72) Inventor: ABBAS, Shabira, S-422 42 Göteborg (SE); STRÖMBOM, Eva, S-431 51 Mölndal (SE); BEMM, Camilla, S-416 61 Göteborg (SE); ÖSTMAN, Äsa, S-412 59 Göteborg (SE); ANNERGREN, Jeanette, S-435 35 Mölnlycke (SE)
(74) Representative: Egeröd, Lisbeth
(86) International application number: PCT/SE2000/001614
(87) International publication number: WO 2001/015649

(56) References cited:
- EP-A1- 0 293 208
- EP-A2- 0 478 011
- WO-A1-97/32612
- US-A- 4 957 810
- US-A- 5 338 766

## Description

### TECHNICAL FIELD

The present invention refers to an absorbent structure in an absorbent article such as a diaper, a pant diaper, an incontinence guard, a sanitary napkin etc. said absorbent structure comprises a compressed foam, which expands upon wetting. The invention further refers to a method of producing an absorbent article containing an absorbent structure according to the invention.

### BACKGROUND

Absorbent articles of the above mentioned kind are intended to absorb body liquids such as urine and blood. Such absorbent articles usually have a liquid pervious topsheet, which during use is facing the wearer's body. They further have a liquid impervious backsheet, e g a plastic film, a plastic coated nonwoven or a hydrophobic nonwoven, and an absorbent structure enclosed between the liquid pervious topsheet and the liquid impervious backsheet. The absorbent structure may comprise two or more layers such as liquid acquisition layer, storage layer and distribution layer.

It is desired that absorbent articles of the above mentioned kind are thin and discrete to use. It is further important that absorbent articles of the above mentioned kind have a high liquid acquisition capacity as well as liquid distributing and liquid storing capacity.

In order to obtain a good liquid acquisition capacity it is important that the liquid acquisition layer has a high momentaneous liquid acquisition capacity. Open, bulky structures with large capillaries have a high momentaneous liquid acquisition capacity and examples of such material are cellulosic fluff pulp of thermomechanic or chemithermomechanic (CTMP) type, chemically stiffened cellulosic fibers, synthetic fiber structures of different kind and porous foam materials.

It is previously known through US-A-3,512,450, EP-A-0 293 208 and EP-A-0 804 913 to use a compressed foam material of regenerated cellulose, e g viscose, as an absorbent structure in an absorbent article of the above mentioned kind, e g a sanitary napkin. The article may then be made very thin and still have a high absorption capacity. The compressed viscose foam expands quickly i the z-direction when liquid is absorbed by the material when wetted. From EP-A-0 293 208 it is further known that such an absorbent structure can contain superabsorbent material.

As storage layer it is commonly used cellulosic fluff pulp with admixture of superabsorbents, i e crosslinked polymers with the ability to absorb liquid several times their own weight. In order to obtain a thin diaper with a maintained total absorption capacity, it is desired to increase the amount of superabsorbent material in the fluff pulp network. In order to make it possible to increase the amount of superabsorbent material it is for example through EP 0 532 002 in absorbent structures known to use superabsorbent material having a good liquid distributing capacity.

WO 97/32612 discloses an absorbent material in the form of a polymeric foam having two distinct zones, which differ in one or more of polymer density, polymer composition etc. There is however no disclosure about the foam containing any superabsorbent material.

Through EP 0 212 618 and EP 0 478 011 it is known to use an absorbent structure that seen in its thickness direction has a gradually increasing concentration of super-absorbent particles, at which a higher concentration of superabsorbent material is localized to the portion of the absorbent structure that during use is placed closest to the liquid impervious backsheet. By using such a structure the risk for gelblocking and an improved liquid distribution is achieved. One problem with such a structure is however that it from processability point of view is difficult to apply the superabsorbent particles so that a gradually increasing particle concentration is achieved in the thickness direction of the structure.

### DESCRIPTION OF THE INVENTION

The problem of providing an absorbent article which is comfortable and discrete to wear, at the same time as it has both a high liquid acquisition capacity and a high liquid distribution capacity, has been substantially eliminated by the present invention.

According to the invention there is provided an absorbent porous structure for use in a diaper, a pant diaper, an incontinence guard, a sanitary napkin etc. and which is provided with a liquid acquisition portion and a liquid storage portion, at which the liquid acquisition portion comprises a compressed polymeric open-cell foam which expands upon wetting, and which is characterized by the liquid acquisition portion and the liquid storage portion being an integrated unit, at which the liquid storage portion comprises a polymeric open-cell foam which may be the same or different from the foam in the liquid acquisition portion, and that the liquid storage portion also contains a superabsorbent material. The amount of superabsorbent material increases gradually in the z-direction of the of the liquid storage portion from the part of the liquid storage portion that is located closest to the liquid acquisition portion to the part of the liquid storage portion that is located furthest away from the liquid acquisition portion. This is advantageous since it reduces the risk for gelblocking further. One advantage of the invention is that the total absorption capacity of the absorbent structure is utilized to a higher degree than if the amount of superabsorbent material is the same in the entire liquid storage layer. In the upper part of the liquid storage layer it is important that the liquid has the ability to be distributed in the longitudinal direction of the structure from the longitudinal mid portion of the structure which coincides with the wetting area, out towards the longitudinal end portions of the absorbent structure. In the lower part of the liquid storage portion it is however mainly important that the absorption capacity is so high that the article has the ability to store so much liquid as possible without leaking.

Thus with the present invention there is obtained an integrated absorbent structure which has a high liquid acquisition capacity as well as a high liquid distribution and liquid storage capacity. An integrated absorbent structure is more advantageous than an absorbent structure comprising different layers since the joining step is eliminated. This involves that the structure is cheaper to manufacture. The problem of having a bad liquid transfer between different layers is further eliminated in an integrated structure. The reason for usually having a worse liquid transfer between different layers than in an integrated structure depends on that it is difficult to achieve a sufficiently good contact between different layers. Another advantage relating to a foam absorbent structure is that it is more flexible and pliable than a fibrous structure. Another advantage with a foam-formed structure is that it is easier to provide a uniform basis weight in the longitudinal and transverse direction of the structure. It has however proved to be difficult to provide a malformed fiber structure having a sufficiently uniform basis weight.

According to one embodiment the portion of the liquid storage portion that as seen in the z-direction is located remote from the liquid acquisition portion only comprises superabsorbent material.

According to an embodiment the superabsorbent material is a porous open-cell foam structure. The advantage of having the superabsorbent material in the form of a foam, is that it is possible to obtain liquid transport between the pores in the superabsorbent material. It is especially important that the superabsorbent material per se has the ability to transport liquid when the amount of superabsorbent is high, i e in the lower part of the liquid storage portion. When the superabsorbent material is in the form of an open-cell foam structure it is also possible that the absorbent material according to the invention only consists of the superabsorbent material. It is also possible that the superabsorbent material comprises a film-forming polymer, which forms a film coating on the pore walls of the foam. Another advantage of having the superabsorbent material in the form of a foam or a film-forming coating, is that such a structure is easy to produce since the difficulties of applying superabsorbent particles in a thickness gradient are eliminated. In such a structure the problem of dusting caused by the smallest superabsorbent particles in the manufacture is eliminated.

The superabsorbent material can for example be based on polyacrylate. It is also possible that the superabsorbent material is based on cellulose or starch.

According to a preferred embodiment the compressed foam structure comprises a regenerated cellulose structure, so called viscose foam. An advantage with a foam of regenerated cellulose is that such a foam when wetted has a very high swelling ability in the z-direction of the structure. This involves that such an article can be very thin before wetting. It is also an advantageous material for articles that are to be shaped into a three-dimensional shape upon wetting, such as for example hump-shaped sanitary napkins. By the fact that the three-dimensional shape appears upon wetting it is possible to produce articles that still are thin and discrete before use.

According to an embodiment the regenerated cellulose foam structure also includes fibers. By incorporating fibrous elements in the regenerated foam structure an improved liquid distributing capacity is achieved.

According to one embodiment the foam structure in the liquid acquisition portion is in dry condition more compressed than the foam structure in the liquid storage portion.

The invention also refers to a method for producing an absorbent structure according to the invention. Such a structure is obtained by shaping a foam material, which is compressed and then dried. After drying the structure a monomer solution of superabsorbent material is added to one of the opposite sides of the foam material, as seen in the z-direction, at which the part of the compressed foam that is wetted by the monomer solution expands. The polymer solution is then polymerized and crosslinked. The structure may optionally be compressed further, after which it is finally dried.

The invention also refers to an absorbent article such as incontinence guard, diaper, pant diaper, sanitary napkin and the like and of the kind comprising a liquid pervious topsheet, a liquid impervious backsheet and an absorbent structure applied therebetween, said absorbent structure containing a structure as disclosed above.

### Description of the drawings

Fig. 1 shows a schematic cross-section through an absorbent structure according to the invention in compressed form, in which the structure in z-direction comprises a liquid acquisition portion and a liquid storage portion.
Fig. 2 shows the absorbent structure according to figure 1 in expanded form.
Fig. 3 shows in a view from above an absorbent article in the form of an incontinence guard.

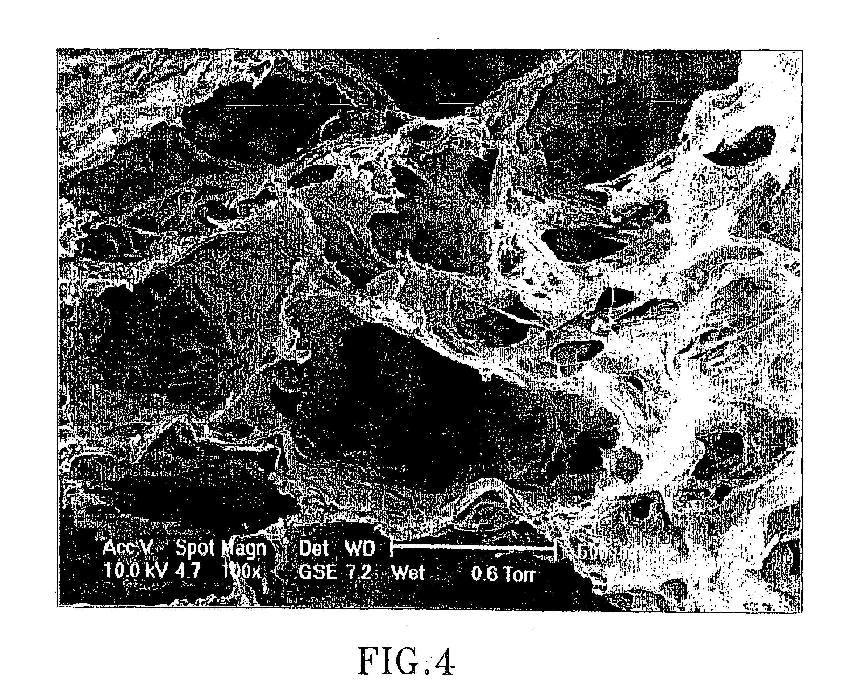

Fig. 4 shows an electron microscope picture (ESEM) of a viscose foam without a superabsorbent film forming coating.

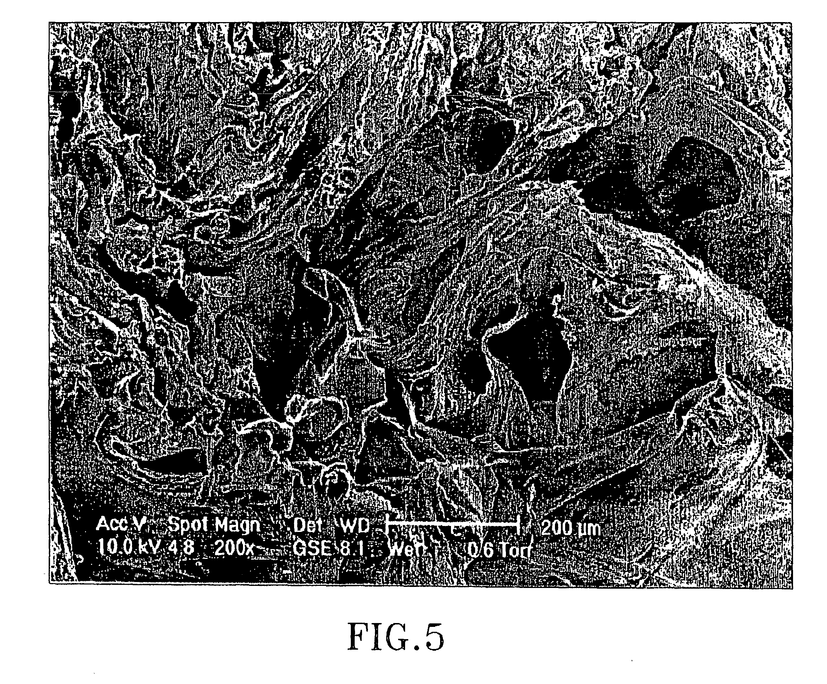

Fig. 5 shows an electron microscope picture (ESEM) of a viscose foam according to the invention with a superabsorbent film forming coating.

### Description of embodiments

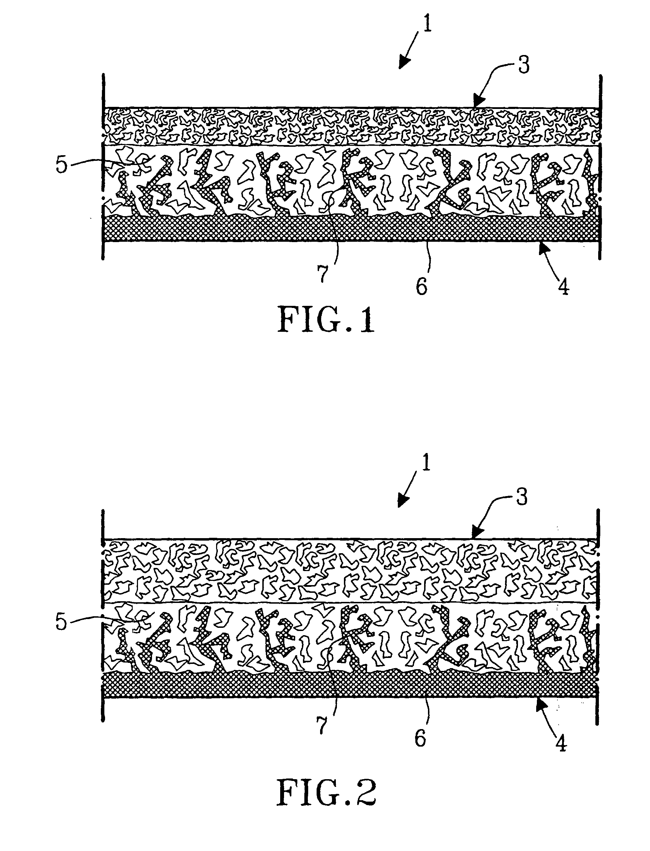

The integrated absorbent structure 1 comprises seen in the in z-direction a liquid acquisition portion 3 and a liquid storage portion 4. The liquid acquisition portion 3 and the liquid storage portion 4 comprises a compressed foam material, which upon liquid contact expands strongly under simultaneous absorption of liquid. Examples of such a foam material is regenerated cellulose. The foam material in the liquid storage portion 4 also comprises a superabsorbent material. When manufacturing the absorbent structure a monomer solution of the superabsorbent is added to one side of the compressed foam. Upon application of the monomer solution to the compressed foam this will expand in the area which is wetted by the monomer solution. This involves that the foam material in the liquid storage portion in dry condition is not so strongly compressed as the foam material in the liquid acquisition portion. The amount of superabsorbent material in the liquid acquisition portion 4 is lowest closest to the liquid acquisition portion and highest furthest away from the liquid acquisition portion. The liquid storage portion 4 in the absorbent structure in figure 1 comprises, as seen in the z-direction, two different portions 5,6. The first portion 5 is located adjacent the liquid acqusition portion 3 and the other portion is located remote from the liquid acquisition portion 3. The first portion 5 comprises the compressed foam, at which the superabsorbent material is placed in the open cells 7 of one part of the porous compressed foam. The other portion 6 consists of only the superabsorbent material. The superabsorbent material is preferably in the form of a foam, but it is also possible that it is in the form of a film-forming gel. The liquid storage portion can of course also consist of only the first portion 5, at which the amount of superabsorbent material decreases towards the liquid acqusition portion.

Figure 2 shows the absorbent structure according to figure 1 in expanded form, i e when the absorbent structure has been wetted during use of the absorbent structure in an absorbent article. The liquid acquisition portion 3 and the liquid storage portion 4 have expanded in the z-direction. The liquid acquisition portion 3 which in dry condition is more compressed than the liquid storage portion 4 has thus expanded more than the liquid storage portion 4.

The amount of superabsorbent material in the liquid storage portion is over 20 weight percent, based on the total weight of the liquid storage portion in dry condition. Preferably the amount of superabsorbent material is over 50 weight percent in the liquid storage portion.

When manufacturing the absorbent porous structure a foam material is formed, which is compressed and then dried. After that a monomer solution of the superabsorbent material is applied to one of the opposite sides of the foam material, as seen in the z-direction. Then the monomer solution is polymerized and crosslinked. The part of the compressed foam which is wetted by the monomer solution is somewhat expanded, while the part of the compressed foam that, as seen in the z-direction, is located furthest away from the side wetted by the monomer solution, is not wetted and therefore not expanded. Since the liquid acquisition portion consists of the part of the compressed foam that, as seen in the z-direction, is located furthest away form the side wetted by the monomer solution, and thus is more compressed, a more rapid liquid acqusition is obtained in the liquid acqusition portion than in the liquid distribution portion. It is also possible to compress the structure further after application of the superabsorbent material.

The monomer solution can be in the form of a solution which upon application on the compressed foam trickles down into the open pores of the compressed foam and forms a filmlike coating. By the fact that a part of the monomer solution trickles into and penetrates part of the open pore system of the compressed foam, there will be obtained a gradually decreasing amount of superabsorbent material in the liquid storage portion of the foam material in the direction away from the side on which the monomer solution has been applied, i e there will be a gradually increasing amount of superabsorbent in the liquid storage portion 4 in the direction away from the liquid acquisition portion 3. The monomer solution can also be in the form of a foamed dispersion solution which after application against one side of the compressed foam is polymerized and crosslinked. The advantage of applying the superabsorbent material in the form of a foamed dispersion solution is that a porous structure is also formed of the superabsorbent material, which involves that the liquid transport in the liquid storage portion is improved. By applying the superabsorbent material in the form of a foamed dispersion solution it is also possible to provide a liquid storage portion which to 100% consists of the superabsorbent material.

It is further possible that the entire absorbent porous structure, i e both the liquid storage portion and the liquid acquisition portion, to 100% consists of a compressed superabsorbent foam. In order to obtain good liquid acquisition properties in the liquid acquisition layer the superabsorbent material in this layer is highly crosslinked. A very highly crosslinked superabsorbent material can not receive so much liquid as a super-absorbent having a lower degree of crosslinking, but on the other side a highly crosslinked superaborbent is better to maintain liquid under pressure. The liquid storage portion in such a structure is crosslinked to a lower degree.

According to a preferred embodiment the foam material is regenerated cellulose, such as viscose, which is a foam comprising a skeleton of cellulose. An advantage of having a foam material of regenerated cellulose is that such a structure has a higher stiffness than foam structures of superabsorbent material based on polyacrylate. Foam materials of regenerated cellulose herewith create firmness to the liquid storage portion.

The principle of making a porous viscose foam is known since long ago and shortly takes place in the following way. Cellulose, usually sulphite pulp, is allowed to swell in sodium hydroxide. Carbon disulphide is added at which the cellulose is successively dissolved. In order to improve the mechanical strength in the material for example cotton fibers may be added. To this cellulose solution there is added and dispersed a salt in the form of sodium sulphate. When then the solution is heated the cellulosed is regenerated (the carbon disulphide is evaporated) and the salt (sodium sulphate ) is dissolved by washing the material with water at which a porous spongelike structure is obtained. The material is dried and compressed if desired

It is also possible to provide an absorbent structure which in its thickness direction has a pore size gradient. In order to provide such a pore size gradient different viscose solutions are used, which are applied on top of each other and then regenerated. Sodium sulphate with different particle sizes is used in the different layers, at which a different pore size of the foam is obtained. By the fact that the different layers are placed on top of each other before they are dry there is achieved an integrated structure, in which the layers partly penetrate into each other.

After regeneration of the cellulose and washing for removing the salt particles the material is dried and compressed to the desired density, which should be in the interval 0.1 to 2.0 g/cm³. The material will upon liquid absorption expand quickly in volume from 2 to 20 times, preferably from 2 to 15 times its volume in compressed condition. The increase of volume at the absorption mainly occurs in the compression direction, i e in the z-direction of the material.

In order to provide a viscose foam containing a certain amount of fibers that are anchored to the pore walls of the foam, fibers can be added to the viscose solution before the foam is shaped. It is also possible to interrupt the dissolving of the cellulose fibers at the addition of the carbon disulphide, so that all fibers are not dissolved. The dissolving can for example be interrupted when 50 weight percent of the cellulose fibers have been dissolved, based on the total dry weight of the cellulose fibers.

The foam may of course be of an optional polymeric material and it is possible to create different mean pore sizes of the respective foam layers by other methods than described above by means of salt crystals of different particle sizes. One such alternative way is to use different types of foaming agents when producing the different foam layers, and which provide different mean pore sizes. Another way is to influence the foaming process in such a way, e g by heating the different layers to different degrees during foaming. In this case it would be possible to use the same foaming agent in the different layers.

The foam materials in the liquid acquisition layer 3 and the liquid storage-layer 4 respectively can be the same. It is however also possible to use different foam materials in the different layers, at which for example a hydrophilicity gradient would be created in the z-direction by having foams of different hydrophilicity/hydrophobicty in the different layers 3 and 4 respectively.

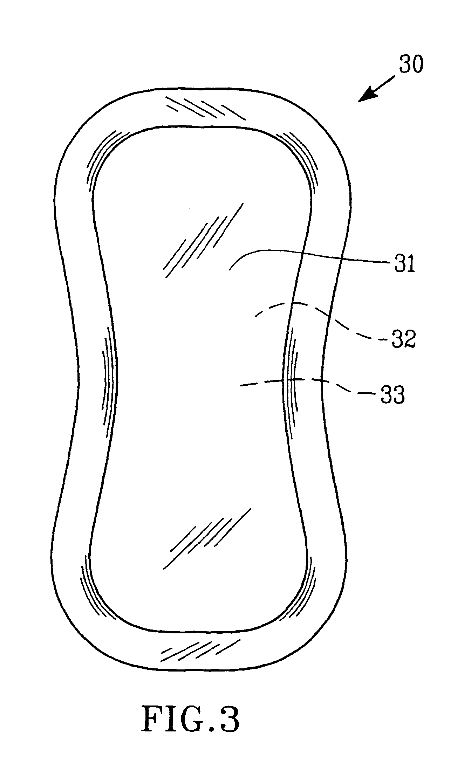

In figure 3 there is shown an absorbent article 30 in the form of an incontinence guard comprising a liquid pervious topsheet 31, a liquid impervious backsheet 32 and absorbent structure 33 according to the invention applied therebetween. The liquid pervious topsheet 31 may be a nonwoven material, e g a spunbond material of synthetic filaments, a thermobonded material, e g a bonded carded fibrous material or a perforated plastic film. The liquid impervious backsheet 32 usually consists of a plastic film, a nonwoven material coated with a liquid impervious material or a hydrophobic nonwoven which resists liquid penetration. The topsheet 31 and the backsheet 32 has a somewhat larger extension in the plane than the absorbent structure 33 and extends outside the edges thereof. The layers 31 and 32 are interconnected within the projecting portions, for example by gluing or welding with heat or ultrasonic.

It is noted that an incontinence guard according to the invention is not limited to embodiment show in the drawing, but the shape of the article as well as its overall design can be varied. The absorbent article can also comprise a diaper, a pant diaper, a sanitary napkin, a bed protection or the like.

The absorbent structure according to the invention may also be arranged over only a part of the total surface of the absorbent body of the absorbent article, e g at the intended wetting area where the discharged body fluid will be deposited and which usually is located towards the front part of the article. The portions of the absorbent body located outside the intended wetting area may then be of another optional absorbent material. It is also possible that the liquid acquisition portion is only located over the area which is intended to be the wetting area, while the liquid storage portion is arranged over the entire surface of the absorbent structure. Such an embodiment is especially preferred when using the absorbent structure in a sanitary napkin. The liquid acquisition portion swells upon discharge of liquid in the z-direction and forms a hump.

The invention is of course not limited to the above mentioned embodiments, but may of course be applied in other embodiments within the scope of the following claims.

## Claims

1. An absorbent porous structure (1) in an absorbent article such as a diaper, a pant diaper, an incontinence guard, a sanitary napkin, said absorbent porous structure comprising a liquid acquisition portion (3) and a liquid storage portion (4), at which the liquid acquisition portion (3) comprises a compressed polymeric open-cell foam which expands upon wetting, wherein the liquid acquisition portion and the liquid storage portion are an integrated unit, at which the liquid storage portion (4) comprises a polymeric open-cell foam which may be the same or different from the foam in the liquid acquisition portion,
**characterized in** and that the liquid storage portion (4) also contains a superabsorbent material, the superabsorbent material being in the form of a film-forming polymer or a foam, the amount of superabsorbent material in the z-direction of the liquid storage portion (4) gradually increases from the part of the liquid storage portion (4) that is located closest the liquid acquisition portion (3) to the part of the liquid storage portion (4) that is located furthest away from the liquid acqusition portion (3).

2. An absorbent structure as claimed in claim 1,
**characterized in that** the part of the liquid storage portion (4) in the z-direction of the absorbent article that is located furthest away from the liquid acquisition portion only consists of the superabsorbent material.

3. An absorbent structure as claimed in claim 1 or 2,
**characterized in that** the superabsorbent material is based on polyacrylate.

4. An absorbent structure as claimed in any of the preceding claims,
**characterized in that** the superabsorbent material is based on cellulose or starch.

5. An absorbent structure as claimed in any of the preceding claims,
**characterized in that** the compressed foam structure is a regenerated cellulose structure.

6. An absorbent structure as claimed in claim 5,
**characterized in that** the regenerated cellulose structure also comprises fibers.

7. An absorbent structure as claimed in any of the preceding claims,
**characterized in that** the foam structure in the liquid acquisition portion in dry condition is more compressed than the foam structure in the liquid storage portion in dry condition.

8. Method of producing an absorbent porous structure (1) in an absorbent article such as a diaper, a pant diaper, an incontinence guard, a sanitary napkin, a wound dressing, a bed protection, said absorbent porous structure comprising a liquid acqusition portion and a liquid storage portion, at which the liquid acquisition portion comprises a compressed polymeric open-cell foam which expands upon wetting, wherein the liquid acquisition portion and the liquid storage portion are an integrated unit, at which the liquid storage portion comprises a polymeric open-cell foam which may be the same or different from the foam in the liquid acquisition portion, **characterized in**
shaping a foam material, which is compressed and then dried, after which a monomer solution of superabsorbent material in the form of a solution or of a foamed dispersion solution is applied to one of opposite sides of the foam material, as seen in the z-direction, at which the part of the compressed foam that is wetted by the monomer solution expands, after which the polymer solution is polymerized and crosslinked and the foam structure is dried.

9. Absorbent article such as an incontinence guard, diaper, pant diaper, sanitary napkin or the like and of the kind comprising a liquid pervious topsheet, a liquid impervious backsheet and an absorbent structure arranged therebetween,
**characterized in that** it contains an absorbent structure as claimed in any of claims 1-7.

## Patentansprüche

1. Absorbierende poröse Struktur (1) in einem absorbierenden Gegenstand, wie einer Windel, einer Hosenwindel, einem Inkontinenzschutz, einer Damenbinde, wobei die absorbierende poröse Struktur einen Flüssigkeitserfassungsabschnitt (3) und einen Flüssigkeitsspeicherabschnitt (4) umfasst, wobei der Flüssigkeitserfassungsabschnitt (3) einen komprimierten, polymeren, offenzelligen Schaum umfasst, der beim Befeuchten expandiert, worin der Flüssigkeitserfassungsabschnitt und der Flüssigkeitsspeicherabschnitt eine integrierte Einheit sind, wobei der Flüssigkeitsspeicherabschnitt (4) einen polymeren, offenzelligen Schaum umfasst, der gleich beschaffen sein kann wie der Schaum im Flüssigkeitserfassungsabschnitt oder sich von diesem unterscheiden kann, **dadurch gekennzeichnet, dass** der Flüssigkeitsspeicherabschnitt (4) auch einen superabsorbierenden Stoff enthält, wobei der superabsorbierende Stoff in Form eines filmbildenden Polymers oder eines Schaums vorliegt, und die Menge des superabsorbierenden Stoffes in der z-Richtung des Flüssigkeitsspeicherabschnitts (4) allmählich von dem Teil des Flüssigkeitsspeicherabschnitts (4), der am nächsten zu dem Flüssigkeitserfassungsabschnitt (3) gelegen ist, zu dem Teil des Flüssigkeitsspeicherabschnitts (4), der am weitesten entfernt von dem Flüssigkeitserfassungsabschnitt (3) gelegen ist, zunimmt.

2. Absorbierende Struktur gemäss Anspruch 1, **dadurch gekennzeichnet, dass** der Teil des Flüssigkeitsspeicherabschnitts (4) in der z-Richtung des absorbierenden Gegenstands, der am weitesten entfernt von dem Flüssigkeitserfassungsabschnitt gelegen ist, nur aus dem superabsorbierenden Stoff besteht.

3. Absorbierende Struktur gemäss Anspruch 1 oder 2, **dadurch gekennzeichnet, dass** der superabsorbierende Stoff auf Polyacrylat beruht.

4. Absorbierende Struktur gemäss einem der vorangehenden Ansprüche, **dadurch gekennzeichnet, dass** der superabsorbierende Stoff auf Cellulose oder Stärke beruht.

5. Absorbierende Struktur gemäss einem der vorangehenden Ansprüche, **dadurch gekennzeichnet, dass** die komprimierte Schaumstruktur eine regenerierte Cellulosestruktur ist.

6. Absorbierende Struktur gemäss Anspruch 5, **dadurch gekennzeichnet, dass** die regenerierte Cellulosestruktur auch Fasern umfasst.

7. Absorbierende Struktur gemäss einem der vorangehenden Ansprüche, **dadurch gekennzeichnet, dass** die Schaumstruktur im Flüssigkeitserfassungsabschnitt in trockenem Zustand stärker komprimiert ist als die Schaumstruktur im Flüssigkeitsspeicherabschnitt in trockenem Zustand.

8. Verfahren zur Herstellung einer absorbierenden porösen Struktur (1) in einem absorbierenden Gegenstand, wie einer Windel, einer Hosenwindel, einem Inkontinenzschutz, einer Damenbinde, einem Wundverband, einem Bettschutz, wobei die absorbierende poröse Struktur einen Flüssigkeitserfassungsabschnitt und einen Flüssigkeitsspeicherabschnitt umfasst, wobei der Flüssigkeitserfassungsabschnitt einen komprimierten, polymeren, offenzelligen Schaum umfasst, der beim Befeuchten expandiert, worin der Flüssigkeitserfassungsabschnitt und der Flüssigkeitsspeicherabschnitt eine integrierte Einheit sind, wobei der Flüssigkeitsspeicherabschnitt einen polymeren offenzelligen Schaum umfasst, der gleich beschaffen sein kann wie der Schaum im Flüssigkeitserfassungsabschnitt oder sich von diesem unterscheiden kann, **dadurch gekennzeichnet, dass** man ein Schaummaterial formt, das komprimiert und dann getrocknet wird, worauf man eine Monomerlösung eines superabsorbierenden Stoffes in Form einer Lösung oder einer geschäumten Dispersionslösung auf die gegenüberliegenden Seiten des Schaummaterials, aus der z-Richtung gesehen, aufbringt, wobei der Teil des komprimierten Schaums, der durch die Monomerlösung befeuchtet wird, expandiert, worauf man die Polymerlösung polymerisiert und vernetzt und die Schaumstruktur trocknet.

9. Absorbierender Gegenstand, wie ein Inkontinenzschutz, eine Windel, eine Hosenwindel, eine Damenbinde oder dergleichen, und von der Art, welche eine flüssigkeitsdurchlässige Oberlage, eine flüssigkeitsundurchlässige Rückenlage und eine dazwischen angeordnete absorbierende Struktur umfasst, **dadurch gekennzeichnet, dass** dieser eine absorbierende Struktur enthält, wie sie in einem der Ansprüche 1 bis 7 beansprucht wird.

## Revendications

1. Structure absorbante poreuse (1) dans un article absorbant tel qu'une couche-culotte, une couche d'apprentissage de la propreté, une protection contre l'incontinence, une serviette hygiénique, ladite structure absorbante poreuse comprenant une section acquisition (3) de liquide et une section stockage (4) de liquide, la section acquisition (3) de liquide comprenant une mousse à cellules ouvertes en polymère comprimé qui se dilate lors du mouillage, la section acquisition de liquide et la section stockage de liquide étant une unité intégrée, la section stockage (4) de liquide comprenant une mousse à cellules ouvertes en polymère qui peut être la même ou différente de la mousse de la section acquisition de liquide,
**caractérisée en ce que** la section stockage (4) de liquide contient aussi un matériau superabsorbant, le matériau superabsorbant se présentant sous la forme d'un polymère formant un film ou d'une mousse, la quantité de matériau superabsorbant dans la direction z de la section stockage (4) de liquide augmente graduellement de la partie de la section stockage (4) de liquide qui est la plus proche de la section acquisition (3) de liquide à la partie de la section stockage (4) de liquide qui est la plus éloignée de la section acquisition (3) de liquide.

2. Structure absorbante selon la revendication 1, **caractérisée en ce que** la partie de la section stockage (4) de liquide, dans la direction z de l'article absorbant, qui est la plus éloignée de la section acquisition de liquide n'est constituée que du matériau superabsorbant.

3. Structure absorbante selon la revendication 1 ou 2, **caractérisée en ce que** le matériau superabsorbant est à base de polyacrylate.

4. Structure absorbante selon l'une quelconque des revendications précédentes, **caractérisée en ce que** le matériau superabsorbant est à base de cellulose ou d'amidon.

5. Structure absorbante selon l'une quelconque des revendications précédentes, **caractérisée en ce que** la structure de mousse comprimée est une structure de cellulose régénérée.

6. Structure absorbante selon la revendication 5, **caractérisée en ce que** la structure de cellulose régénérée comprend aussi des fibres.

7. Structure absorbante selon l'une quelconque des revendications précédentes, **caractérisée en ce que** la structure de mousse de la section acquisition de liquide, à l'état sec, est plus comprimée que la structure de mousse de la section stockage de liquide à l'état sec.

8. Procédé de production d'une structure absorbante poreuse (1) dans un article absorbant tel qu'une couche-culotte, une couche d'apprentissage de la propreté, une protection contre l'incontinence, une serviette hygiénique, un pansement, une alèse, ladite structure absorbante poreuse comprenant une section acquisition de liquide et une section stockage de liquide, la section acquisition de liquide comprenant une mousse à cellules ouvertes en polymère comprimé qui se dilate lors du mouillage, la section acquisition de liquide et la section stockage de liquide étant une unité intégrée, la section stockage de liquide comprenant une mousse à cellules ouvertes en polymère qui peut être la même ou différente de la mousse de la section acquisition de liquide, **caractérisé par** le fait de former un matériau mousse, qui est comprimé puis séché, après quoi une solution de monomère de matériau superabsorbant, sous la forme d'une solution ou d'une solution-dispersion que l'on a fait mousser, est appliquée à l'un des côtés opposés du matériau mousse, vus dans la direction z, où la partie de la mousse comprimée qui est mouillée par la solution de monomère se dilate, après quoi la solution de polymère est polymérisée et réticulée puis la structure de mousse est séchée.

9. Article absorbant tel qu'une protection contre l'incontinence, une couche-culotte, une couche d'apprentissage de la propreté, une serviette hygiénique ou similaire et du type comprenant une feuille avant perméable aux liquides, une feuille arrière imperméable aux liquides et une structure absorbante placée entre elles, **caractérisé en ce qu'**il contient une structure absorbante conforme à l'une quelconque des revendications 1 à 7.
